# EUROPEAN PATENT APPLICATION

(11) **EP 1 769 834 A2**
(43) Date of publication of application: **04.04.2007**
(21) Application number: 06255060.3
(22) Date of filing: 29.09.2006
(51) Int. Cl.: B01D 7/00, B01D 7/02, B01D 19/00

(54) **Methods for providing oxidatively stable ophthalmic compositions**

(30) Priority: 30.09.2005 US 241313
(71) Applicant: Johnson and Johnson Vision Care, Inc., Jacksonville, FL 32256 (US)
(72) Inventor: Alli, Azaam, Jacksonville, Florida 32277 (US); Mahadevan, Shivkumar, Orange Park, Florida 32003 (US)
(74) Representative: Fisher, Adrian John

(57) **Abstract**

This invention relates to a process for improving the stability of an ophthalmically compatible solution comprising at least one oxidatively unstable ophthalmic compound.

## Description

### FIELD OF THE INVENTION

The present invention relates to methods for providing ophthalmic compounds that display oxidative stability, during processing, autoclaving, packaging, shipping or storage.

### BACKGROUND OF THE INVENTION

Therapeutic agents for topical administration to the eye are generally formulated in either a liquid or gel form and must be kept sterile until administration. Accordingly, ophthalmic therapeutic agents are either packaged asceptically, which is cumbersome and expensive or are heat sterilized. Unfortunately, many therapeutic agents are not oxidatively stable, especially at elevated temperatures.

EDTA has been used to improve the stability of certain therapeutic agents during autoclaving. However, there remains a need for processes capable of stabilizing unstable therapeutic agents that are susceptible to oxidative degradation.

### SUMMARY OF THE INVENTION

The present invention relates to a method comprising removing at least about 80% oxygen from an ophthalmically compatible solution comprising at least one oxidatively unstable ophthalmic compound.

### DETAILED DESCRIPTION OF THE INVENTION

The present invention comprises, consists of and consists essentially of stabilizing at least one oxidatively unstable ophthalmic compound dissolved in an ophthalmically compatible solution by removing at least about 80% oxygen from said ophthalmically compatible solution comprising at least one oxidatively unstable ophthalmic compound. In some embodiments at least about 90% of said oxygen is removed. In some embodiments at least about 95% of said oxygen is removed and in still other embodiments at least about 99% of said oxygen is removed.

As used herein, oxidatively unstable ophthalmic compound ("OUOC") is any therapeutic agent which shows greater than 10% degradation when autoclaved in solution with at least one oxidative catalyst, but shows less than 10% degradation when autoclaved under the same conditions without said at least one oxidative catalyst. Oxidative instability may be measured by forming a solution of 3 ml packing solution containing 25 ppm of the therapeutic agent to be evaluated, and exposing the solution, with and without oxidative catalysts (100 ppm Cu₂O and 100 ppm FeSO₄) to autoclave conditions (120°C for 20 minutes).

Examples of OUOC include oxidatively unstable pharmaceutical and nutraceutical compounds. In one embodiment the OUOC comprises at least one pharmaceutically active amines. In one embodiment the at least one OUOC comprises at least one tertiary cyclic amine. In another embodiment the at least one OUOC comprises at least one tertiary cyclohexyl amine. In another embodiment the OUOC comprises at least one therapeutic agent selected from as acycylovir, adrenalone, aminocaproic acid, amoxicillin, amotriphene, amoxecaine, amodiaquin, antazoline, atrophine, betaxolol, bupivacaine, carbachol, carteolol, chlorampenicol, chlortetracycline, corynathine, cromalyn sodium, cyclopentolate, demecarium, dexamethasone, dichlorphenamide, dibutoline, diclophenac, dipivefrin, ephedrine, erythromycin, ethambutol, eucatropine, fluoromethalone, gentamycin, gramicidin, homatropine, indomethacin, ketotifen, levallorphan, levobunolol, levocabastine, lidocaine, lignocaine, lomefloxacin, medrysone, mepivacaine, methazolamide, naphazoline, natamycin, natamycin, neomycin, noradrenaline, ofloxacin, oxybuprocaine, oxymetazoline, pheniramine, phenylephrine, physostigmine, pilocarpine, polymyxin B, prednisolone, proparacaine, pyrilamine, scopolamine, sorbinil, sulfacetamide, tamoxifen, tetracaine, tetracycline, tetrahydozoline, timolol, trifluridine, tropicamide, vidarabine, and salts and mixtures thereof. Examples of nutriceutical compounds include vitamins and supplements such as vitamins A, D, E, lutein, zeaxanthin, lipoic acid, flavonoids, ophthalmicially compatible fatty acids, such as omega 3 and omega 6 fatty acids, combinations thereof, combinations with pharmaceutical compounds and the like. In yet another embodiment the OUOC comprises at least one therapeutic agent selected from ketotifen fumarate, nor ketotifen fumarate, 11-dihydro-11-(1-methyl-4-piperidinylidene)-5H-imidazo[2,1-b][3]benzazepine-3-carboxaldehyde (CAS# 147084-10-4, olapatadine and mixtures thereof. In yet another embodiment the OUOC comprises at least one therapeutic agent selected from ketotifen fumarate, 11-dihydro-11-(1-methyl-4-piperidinylidene)-5H-imidazo[2,1-b][3]benzazepine-3-carboxaldehyde (CAS# 147084-10-4 and mixtures thereof.

The concentration of the OUOC in the ophthalmically compatible solutions of the present invention may range from about 10 ppm to about 100,000 ppm, in some embodiments from about 10 to about 10,000 ppm, in some embodiments from about 10 to about 1,000 ppm and some embodiments from about 10 to about 500 ppm.

The process of the present invention comprises removing at least 80% oxygen from the ophthalmically compatible solution comprising at least one OUOC. In some embodiments the amount of oxygen removed is at least about 90%, at least about 95%, and even at least about 99%. The oxygen removal is conducted prior to heat sterilization, such as autoclaving. For example, oxygen removal may be conducted either before or after the OUOC is added to the ophthalmically compatible solution. If the oxygen removal is conducted before the OUOC is added to the ophthalmically compatible solution, the solution should be kept under conditions sufficient to prevent oxygen from being introduced into the ophthalmically compatible solution during mixing and autoclaving.

The oxygen may be removed by a number of methods including sparging, alternating freezing and thawing cycles, vacuum removal, vacuum removal in combination with agitation, combinations thereof and the like.

When sparging is used at least one inert gas which is capable of displacing oxygen is bubbled through the ophthalmically compatible solution under conditions suitable to remove the desired amount of oxygen. Suitable inert gasses include nitrogen, argon, helium, combinations thereof and the like. Suitable sparging conditions include volumes of ophthalmically compatible solution of at least about 1L, between about 1 and about 6L; and between about 1 and about 4L. In some embodiments a volume of about 2L is desirable. The inert gas flow rate may be selected based upon the selected volume and desired sparging time. So, for example, higher volumes may require higher inert gas flow rates, sparging times or a combination of both. Suitable inert gas flow rates include from about 10 to about 1000 standard cubic centimeter per minute (SCCM). In some embodiments an inert gas flow rate of about 370 SCCM is desirable.

Sparging times may vary based upon the other conditions as described above. Suitable sparging times include at least about 5 minutes, from about 5 minutes to 24 hours, from 5 minutes to 12 hours and in some embodiments at least about 8 hours.

Any temperature can be used for sparging so long as the ophthalmically compatible solution remains a liquid and the OUOC is soluble in the ophthalmically compatible solution at the selected temperature. Temperatures between about 0 to about 40°C may be used in some embodiments.

Alternatively the oxygen may be removed by freeze thaw degassing. Suitable pressures for freeze thaw degassing include those less than about 660 mmHg. For degassing an aqueous solution at room temperature, pressures between 660 and 760mm Hg are desirable. Gentle agitation or sonication increases the efficiency of the process.

When sparging is used the process of the present may also include agitation which may be provided by any known method such as, but not limited to sonication, stirring, rolling, shaking, combinations thereof and the like.

Freeze thaw degassing comprises freezing the ophthalmically compatible solution to form a solid and then thawing the solid. This process may be repeated.

Alternatively, the ophthalmically compatible solution may be exposed to degas conditions below its vapor pressure. For example, if an ophthalmically compatible solution's boiling point at a given pressure is 20°C, the solution is cooled to less than about 20°C prior to evacuating the system to the desired pressure. When placed under a vacuum, all dissolved gases are removed from the ophthalmically compatible solution, while little or none of the other components (solvents or solutes) are displaced. Once the gas bubbles cease to escape from the ophthalmically compatible solution, the system is placed under a positive pressure of an inert gas such as nitrogen or argon, and allowed to warm up to ambient temperature.

The degassing cycle described above may be repeated once or more depending on how sensitive the ophthalmically compatible solution is to oxidative degradation, with solutions which are more susceptible to degradation being subjected to more cycles.

The ophthalmically compatible solution may be formed from any suitable ophthalmically compatible carrier. Suitable carriers include water, saline solution, mineral oil, petroleum jelly, water soluble solvents, such as C₁₅₋₂₀ alcohols, C₁₅₋₂₀ amides, C₁₅₋₂₀ alcohols substituted with zwitterions, vegetable oils or mineral oils comprising from 0.5 to 5% by weight hydroxyethylcellulose, ethyl oleate, carboxymethylcellulose, polyvinyl-pyrrolidone and other non-toxic water-soluble polymers for ophthalmic uses, such as, for example cellulose derivatives, such as methylcellulose, alkali metal salts of carboxy-methylcellulose, hydroxymethylcellulose, methylhydroxypropyl-cellulose, hydroxypropylcellulose, chitosan and scleroglucan, acrylates or methacrylates, such as salts of poly(acrylic acid) or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as poloxamers, e.g. Poloxamer F127, polyvinyl alcohol, polyvinyl methyl ether, polyethylene oxide, cross-linked poly(acrylic acid), such as neutral Carbopol, or mixtures of those polymers. Preferred carriers are water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol, or mixtures thereof. The concentration of the carrier is, for example, from 0.1 to 100000 times the concentration of the active ingredient combinations thereof and the like. When the ophthalmically compatible solution is an eye drop, suitable carriers include water, pH buffered saline solution, mixtures thereof and the like.

The ophthalmically compatible solution of the present invention may also be used as the packaging or storage solution for an ophthalmic device, such as a contact lens. When the ophthalmically compatible solution is used as a packaging solution for a contact lens the carrier comprises a buffered saline solution. Any contact lens could be packaged with the ophthalmically compatible solution of the present invention, including conventional and silicone hydrogel contact lenses, such as but not limited to commercially available hydrogel formulations such as etafilcon, polymacon, vifilcon, genfilcon A, lenefilcon A, galyfilcon, senofilcon, balafilcon, lotrafilcon A, lotrafilcon B and the like.

The ophthalmically compatible solution of the present invention may further comprise additional components such as antioxidants, demulcents, antibacterial agents, solubilizers, surfactants, buffer agents, tonicity adjusting agents, chelating agents, preservatives, wetting agents, thickeners, stabilizers, combinations thereof and the like. An example of a suitable stabilizer includes EDTA. The ophthalmically compatible solution of the present invention are ophthalmically compatible, and have a pH between about 5 and about 9, in some embodiments between about 6 to about 8 is desired.

The ophthalmically compatible solution of the present invention may be formed by mixing the OUOC and any additional components with the selected carrier. When a liquid composition, such as an eye drop or packaging solution for a contact lens, the OUOC and any additional components are dissolved in the carrier.

It is generally desirable that the shelf life of the ophthalmically compatible solution be in excess of about 6 months, and in some instances greater than about 1 year, or even more than about 2 years. During the shelf life of the ophthalmically compatible solution it is desirable that at least about than 80% of the original concentration of the OUOC remains, and in some embodiments greater than about 90%.

The ophthalmically compatible solution of the present invention may in some embodiments further comprise at least one electron rich polymer. Suitable electron rich polymers are water-soluble, comprise at least one group with a free electron pair, have a weight average molecular weight, Mw, between about 1000 and about 2,000,000, and are substantially free from transition metal containing species. In some embodiments the electron rich polymers are substantially free from copper and iron containing species. As used herein, "substantially free from" means that transition metal containing species are present in the electron rich polymer in amounts which are insufficient to cause further degradation of the OUOC. Preferably the transition metal containing species are present in the electron rich polymer in amounts less than about 100 ppm, in some embodiments less than about 50 ppm and in some embodiments less than about 20 ppm.

As used herein, water soluble means that the selected electron rich polymer does not precipitate or form visible gel particles at the concentrations selected and across the temperatures and pH regimes common for manufacturing, sterilizing and storing ophthalmic solutions.

For purposes of the invention, the molecular weight is determined using a gel permeation chromatograph with a 90° light scattering and refractive index detectors. Two columns of PW4000 and PW2500, a methanol-water eluent of 75/25 wt/wt adjusted to 50mM sodium chloride and a mixture of polyethylene glycol and polyethylene oxide molecules with well defined molecular weights ranging from 325,000 to 194 are used.

Suitable examples of electron rich polymers include polymers comprising esters, acids, amines, carbonates, carboxylates, thiols, lactates, amides, carbamates, phosphates, nitriles, lactams, and combinations thereof. Polymers which do not have groups with at least one free electron pair, such as polymers comprising only ether groups, alcohol groups or combinations thereof are not electron rich polymers are defined herein. A wide concentration of electronic donating groups may be included, however, the higher the concentration of electron donating groups, the less electron rich polymer will need to be used. Specific examples include homopolymers and random or block copolymers of methacrylic acid, acrylic acid, itaconic acid, fumaric acid, maleic acid, vinylpyrollidone, vinylmethacetimide, combinations thereof and the like. More specific examples include poly(acrylic acid), poly(vinylpyrollidone) and poly(vinylmethylacetamide) and combinations thereof and the like. In one embodiment the electron rich polymer comprises poly(acrylic acid).

The electron rich polymer is present in the ophthalmically compatible solution in stabilizing effective amounts. A stabilizing effective amount will vary depending upon the OUOC, the concentration of the OUOC and the concentration of other components in the ophthalmically compatible solution, but generally stabilizing effective amounts are those sufficient to provide at least about a 5% improvement in stability. Suitable amounts of electron rich polymer include between about 10 and about 5,000 ppm, in some embodiments between about 100 and about 5,000 ppm, in some embodiments between about 500 and about 3,000 ppm.

These examples do not limit the invention. They are meant only to suggest a method of practicing the invention. Those knowledgeable in contact lenses as well as other specialties may find other methods of practicing the invention. However, those methods are deemed to be within the scope of this invention.

### Examples 1

A buffer solution was formed by dissolving 8.3 gm NaCl (from Sigma Aldrich), 9.1 gm boric acid (from Mallinckrodt) and 1 gm sodium borate (from Mallinckrodt) in 1L deionized water (from Milli Q). The resulting solution had a pH of 7.65. Ketotifen fumarate (from Sigma Aldrich) was added to prepare a solution of approximately 80 ppm in the buffer solution. The ketotifen solution (3 mL) was placed in vials, autoclaved for the number of cycles shown in Table 1, below and analyzed as a function of autoclave cycle (3 replicate per autoclave cycle) using HPLC using an HP1100 and an Agilent Zorbax Eclipse XDB-C18 and Rapid Resolution HT 50 x 4.6 mm x 1.8 µ column and the following conditions:

| | |
|---|---|
| Detector Wavelength : | 299 nm |
| Flow rate: | 1.0 mL/min |
| Injection Volume: | 3 µL |

### Mobile Phase:

- Eluent A:: 17 % acetonitrile in 0.025 M dihydrogen potassium phosphate buffer
0.2 % triethylamine, 0.13 % o-phosphoric acid
- Eluent B:: 50 % acetonitrile in 0.025 M dihydrogen potassium phosphate buffer
0.2 % triethylamine, 0.13 % o-phosphoric acid

| Time (min) | Eluent A (%) | Eluent B (%) |
|---|---|---|
| 0 | 100 | 0 |
| 5 | 100 | 0 |
| 20 | 0 | 100 |
| 21 | 100 | 0 |
| 25 | 100 | 0 |

The results are shown in Table 1, below.

**Table 1**

| Autoclave Cycle | % ketotifen |
|---|---|
| 0 | 100 |
| 1 | 0 |
| 2 | 0 |
| 3 | 0 |

The results in Table 1 clearly show that even a single autoclave cycle has a substantial detrimental effect on the ketotifen concentration in a buffer solution.

### Example 2

Example 1 was repeated, except that the buffer solution was sparged (with nitrogen) overnight (~12 hrs) at about 370 standard cubic centimeter per minute (SCCM) and subsequently transferred to a nitrogen box (<0.5 % oxygen). The ketotifen solution of about 90 ppm was prepared and placed in vials as described above, but in nitrogen box. The vials were autoclaved and analyzed as described in Example 1. The results are shown in Table 2, below.

**Table 2**

| Autoclave Cycle | % ketotifen |
|---|---|
| 0 | 100 |
| 1 | 98 |
| 2 | 98 |
| 3 | 98 |

Comparing the results in Table 2 to those in Table 1, it is clear that sparging the buffer solution and maintaining the ketotifen solution under nitrogen significantly improved (from 0 to 98%) the ketotifen stability.

### Example 3 and 4

Examples 1 and 2 was repeated, except that 10 gm of poly(acrylic acid) (PAA, Mw, 225,000, from Polysciences, Inc., 20 % in water) was added to the buffer solution. The vials were autoclaved and analyzed as described in Example 1. The results are shown in Table 3, below.

**Table 3**

| Autoclave Cycle | Example 3 % ketotifen Unsparged | Example 4 % ketotifen Nitrogen Sparged |
|---|---|---|
| 0 | 100 | 100 |
| 1 | 94 | 99 |
| 2 | 81 | 99 |
| 3 | 66 | 98 |

The results for Example 3 (inclusion of an electron rich polymer, such as PAA, with no sparging) are far superior to those of Example 1 (no electron rich polymer, no sparging). However, even with an electron rich polymer some ketotifen is lost after multiple autoclaving cycles. However, Example 4 (electron rich polymer and sparging) shows improved stability of the oxidatively unstable ophthalmic composition. Thus the foregoing examples clearly show that removing oxygen from the ophthalmically compatible solution significantly improves the stability of an oxidatively unstable ophthalmic composition, like ketotifen fumarate.

## Claims

1. A process comprising removing at least 80% oxygen from an ophthalmically compatible solution comprising at least one oxidatively unstable ophthalmic compound.

2. The process of claim 1 wherein at least 90% of said oxygen is removed.

3. The process of claim 1 wherein at least 95% of said oxygen is removed.

4. The process of claim 1 wherein at least 99% of said oxygen is removed.

5. The process of claim 1 wherein said removing step is accomplished via a method selected from the group consisting of sparging, alternating freezing and thawing cycles, vacuum removal and vacuum removal in combination with agitation and combinations thereof.

6. The process of claim 5 wherein agitation is provided via sonication, stirring, rolling, shaking and combinations thereof.

7. The process of claim 5 wherein said removing step is accomplished by sparging.

8. The process of claim 7 wherein said sparging is conducted using an inert gas capable of displacing oxygen.

9. The process of claim 8 wherein said inert gas is selected from the group consisting of nitrogen, argon, helium and mixtures thereof.

10. The process of claim 9 wherein said sparging is conducted using conditions comprising a volume of ophthalmically compatible solution of about 2 L a flow rate of 370 SCCM (standard cubic centimeter per minute) and a sparging time of at least about 8 hours.

11. The process of any preceding claim wherein said conditions further comprise a temperature from 0 to 40°C and a pressure of less than 660 mmHg.

12. The process of any one of claims 1 to 10 wherein said conditions further comprise room temperature and pressures between 660 and 760mm Hg.

13. The process of any preceding claim wherein said ophthalmically compatible solution further comprises at least one stabilizer.

14. The process of claim 13 wherein said stabilizer comprises at least one electron rich polymer.

15. The process of claim 13 wherein said stabilizer comprises EDTA.
